# EUROPEAN PATENT APPLICATION

(11) **EP 3 919 007 A1**
(43) Date of publication of application: **08.12.2021**
(21) Application number: 21177252.0
(22) Date of filing: 01.06.2021
(51) Int. Cl.: A61B 17/32

(54) **ULTRASONIC TRANSDUCER CASING, ULTRASONIC TRANSDUCER ASSEMBLY, AND ULTRASONIC SURGICAL INSTRUMENT**

(30) Priority: 02.06.2020 US 202063033393 P; 05.05.2021 US 202117308936
(71) Applicant: Covidien LP, Mansfield, MA 02048 (US)
(72) Inventor: COWLEY, Matthew, S, Boulder, CO Colorado 80301 (US)
(74) Representative: Maschio, Antonio

(57) **Abstract**

A casing of an ultrasonic transducer assembly incudes a first portion, a second portion, and at least one seal member. The first portion includes a ridge disposed on a first mating surface of the first portion. The second portion includes a trough defined within a second mating surface of the second portion and configured to receive the ridge of the first portion. The at least one seal member extends from the first mating surface of the first portion inwardly of the ridge and/or the second mating surface of the second portion inwardly of the trough. The first mating surface of the first portion and the second mating surface of the second portion are configured to be fused to one another. The fusion and the at least one seal member establish a hermetic seal between the first and second mating surfaces.

## Description

### CROSS-REFERENCE TO RELATED APPLICATIONS

This application claims the benefit of, and priority to, U.S. Provisional Patent Application No. 63/033,393, filed on June 2, 2020, the entire contents of which are hereby incorporated herein by reference.

### FIELD

The present disclosure is generally related to ultrasonic surgical instruments and more particularly to an ultrasonic transducer casing and ultrasonic transducer assembly for an ultrasonic surgical instrument.

### BACKGROUND

Ultrasonic surgical instruments utilize ultrasonic energy, i.e., ultrasonic vibrations, to treat tissue. More specifically, ultrasonic surgical instruments utilize mechanical vibration energy transmitted at ultrasonic frequencies to coagulate, cauterize, fuse, seal, cut, and/or desiccate tissue to effect hemostasis.

Ultrasonic surgical instruments typically employ a transducer coupled to a housing of the ultrasonic surgical instrument and configured to produce ultrasonic energy for transmission along a waveguide to an end effector of the ultrasonic surgical instrument that is designed to treat tissue with the ultrasonic energy. The transducer may be driven by an ultrasonic generator that is on-board, e.g., on or within the housing of the ultrasonic surgical instrument, or remotely disposed, e.g., as a set-top box connected to the ultrasonic surgical instrument via a cable. The end effector of the ultrasonic surgical instrument may include a blade that receives the ultrasonic energy from the waveguide for application to tissue and a jaw member configured to clamp tissue between the blade and the jaw member to facilitate treatment thereof.

### SUMMARY

The present disclosure relates to an ultrasonic transducer casing, ultrasonic transducer assembly, and ultrasonic surgical instrument wherein the casing includes a first portion and a second portion fused to one another to create a sealed enclosure.

In one aspect, the present disclosure provides a casing of an ultrasonic transducer assembly including a first portion, a second portion, and at least one seal member. The first portion includes a ridge disposed on a first mating surface of the first portion. The second portion includes a trough defined within a second mating surface of the second portion that is configured to receive the ridge of the first portion. The at least one seal member extends from the first mating surface of the first portion inwardly of the ridge and/or the second mating surface of the second portion inwardly of the trough. The first mating surface of the first portion and the second mating surface of the second portion are fused to one another. The fusion and the at least one seal member establish a hermetic seal between the first and second mating surfaces.

In aspects, the at least one seal member may be engaged with the first mating surface or the second mating surface via overmolding.

In aspect, the at least one seal member may include one seal member extending from the first mating surface and another seal member extending from the second mating surface.

In aspects, the first portion further may include a first distal opening configured to receive a first portion of an ultrasonic horn of an ultrasonic transducer assembly and the second portion may further include a second distal opening configured to receive a second portion of the ultrasonic horn.

In aspects, the casing may further include a first distal seal member surrounding the first distal opening and a second distal seal member surrounding the second distal opening.

In aspects, the first and second distal seal members may be engaged with the first and second portions, respectively, via overmolding.

In aspects, one of the first distal seal member or the second distal seal member may be integrally formed with the seal member.

In aspects, the first and second distal seal members may be configured to establish a hermetic seal about the ultrasonic horn.

In aspects, the first portion and/or the second portion further may include at least one pass-through electrical contact.

In aspects, the first portion and/or the second portion further may include a bumper disposed therein towards a proximal end thereof to support a proximal end portion of an ultrasonic transducer assembly.

In another aspect, the present disclosure provides an ultrasonic transducer assembly of an ultrasonic surgical instrument including a piezoelectric stack, an ultrasonic horn, and a casing. The ultrasonic horn is secured to and extends distally from the piezoelectric stack. The ultrasonic horn includes a body and a nose extending distally from the body. The casing is disposed about the piezoelectric stack and at least a portion of the body of the ultrasonic horn with the nose extending distally from the casing. The casing includes a first portion, a second portion, and at least one seal member. The first portion includes a ridge disposed on a first mating surface of the first portion. The second portion includes a trough defined within a second mating surface of the second portion that is configured to receive the ridge of the first portion. The at least one seal member extends from the first mating surface of the first portion inwardly of the ridge and/or the second mating surface of the second portion inwardly of the trough. The first mating surface of the first portion and the second mating surface of the second portion are fused to one another. The fusion and the at least one seal member establish a hermetic seal between the first and second mating surfaces.

In aspects, the at least one seal member may be engaged with the first mating surface or the second mating surface via overmolding.

In aspects, the at least one seal member may include one seal member extending from the first mating surface and another seal member extending from the second mating surface.

In aspects, the first portion further may include a first distal opening configured to receive a first portion of the ultrasonic horn and the second portion further may include a second distal opening configured to receive a second portion of the ultrasonic horn.

In aspects, the first overmold and the second overmold may establish a hermetic seal.

In aspects, the casing may further include a first distal seal member surrounding the first distal opening and a second distal seal member surrounding the second distal opening.

In aspects, the first and second distal seal members may be engaged with the first and second portions, respectively, via overmolding.

In aspects, one of the first distal seal member or the second distal seal member may be integrally formed with the seal member.

In aspects, the first and second distal seal members may be configured to establish a hermetic seal about the ultrasonic horn.

In aspects, the first portion and/or the second portion further may include a bumper disposed threrein towards a proximal end thereof to support the piezoelectric stack.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

Embodiments of the presently disclosed ultrasonic transducer casing, ultrasonic transducer assembly, and ultrasonic surgical instrument are described in detail with reference to the drawings, in which like reference numerals designate identical or corresponding elements in each of the several views.
FIG. 1 is a side, perspective view of an ultrasonic surgical instrument provided in accordance with the present disclosure;
FIG. 2 is an enlarged, side, longitudinal, cross-sectional view of a proximal portion of the ultrasonic surgical instrument of FIG. 1;
FIG. 3 is an enlarged, perspective view of an ultrasonic transducer assembly of the ultrasonic surgical instrument of FIG. 1;
FIG. 4 is an enlarged, e5xploded, perspective view of a casing of the ultrasonic transducer assembly of FIG. 3;
FIG. 5A is an enlarged, cross-sectional side view taken along a sectional line 5-5 of FIG. 3;
FIG. 5B is an enlarged, cross-sectional side view taken along a sectional line 5-5 of FIG. 3 illustrating only the casing; and
FIG 5C is an enlarged, cross-sectional view taken along a sectional line 6-6 of FIG. 3 illustrating only the casing.

### DETAILED DESCRIPTION

As used herein, the term "distal" refers to that portion of the instrument or component thereof that is farther from the operator (whether a clinician or a surgical robot), while the term "proximal" refers to that portion of the instrument or component thereof that is closer to the user operator.

As used herein, the term "clinician" refers to a doctor, nurse, surgeon, or other care provider and may include support personnel and surgical robots operated by a clinician. In the following description, well-known functions, or construction are not described in detail to avoid obscuring the disclosure in unnecessary detail.

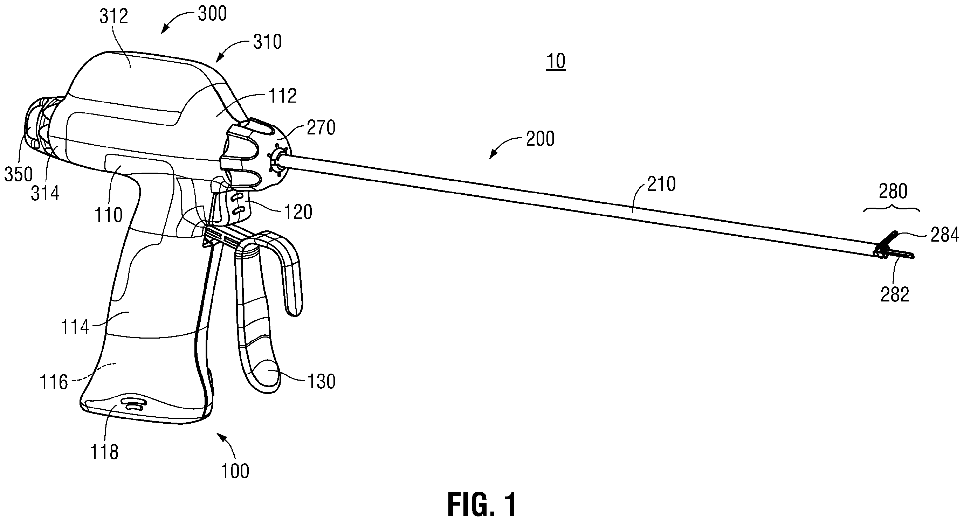

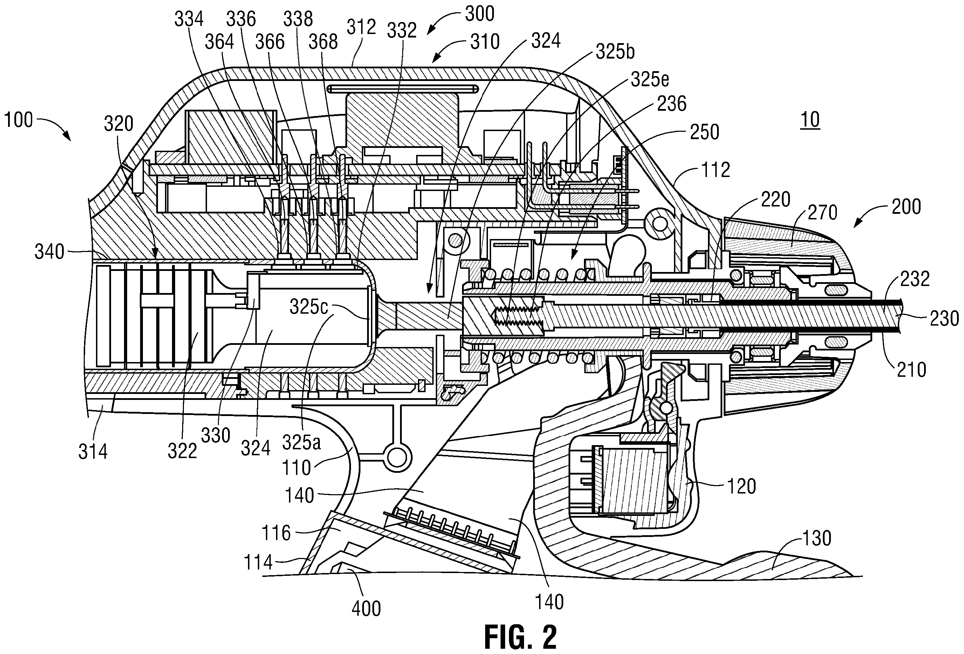

Referring to FIGS. 1 and 2, an ultrasonic surgical instrument provided in accordance with the present disclosure is shown generally identified by reference numeral 10. Ultrasonic surgical instrument 10 includes a handle assembly 100 and an elongated assembly 200 extending distally from handle assembly 100. Handle assembly 100 includes a housing 110 defining a body portion 112 and a fixed handle portion 114. Handle assembly 100 further includes an activation button 120 and a clamp trigger 130.

Body portion 112 of housing 110 is configured to support an ultrasonic transducer and generator assembly ("TAG") 300 including a generator assembly 310 and an ultrasonic transducer assembly 320. TAG 300 may be permanently engaged with body portion 112 of housing 110 or removable therefrom. Generator assembly 310 includes a housing 312 configured to house the internal electronics of generator assembly 310, and a cradle 314 configured to rotatably support ultrasonic transducer assembly 320. Alternatively, generator assembly 310 may be remotely disposed and coupled to ultrasonic surgical instrument 10 by way of a surgical cable. TAG 300 is described in greater detail below.

Fixed handle portion 114 of housing 110 defines a compartment 116 configured to receive a battery assembly 400 and a door 118 configured to enclose compartment 116. An electrical connection assembly 140 is disposed within housing 110 of handle assembly 100 and serves to electrically couple activation button 120, generator assembly 310 of TAG 300, and battery assembly 400 with one another when TAG 300 is supported on or in body portion 112 of housing 110 and battery assembly 400 is disposed within compartment 116 of fixed handle portion 114 of housing 110, thus enabling activation of ultrasonic surgical instrument 10 in response to depression of activation button 120. In embodiments where generator assembly 310 is remote from ultrasonic surgical instrument 10, battery assembly 400 and the configuration of fixed handle portion 114 for receiving battery assembly 400 need not be provided, as generator assembly 310 may be powered by a standard wall outlet or other power source.

Referring still to FIGS. 1 and 2, elongated assembly 200 of ultrasonic surgical instrument 10 includes an outer drive sleeve 210, an inner support sleeve 220 disposed within outer drive sleeve 210, a waveguide 230 extending through inner support sleeve 220, a drive assembly 250, a rotation knob 270, and an end effector 280 including a blade 282 and a jaw 284. A proximal portion of outer drive sleeve 210 is operably coupled to clamp trigger 130 of handle assembly 100 via drive assembly 250, while a distal portion of outer drive sleeve 210 is operably coupled to jaw 284. As such, clamp trigger 130 is selectively actuatable to thereby move outer drive sleeve 210 about inner support sleeve 220 to pivot jaw 284 relative to blade 282 of end effector 280 from a spaced-apart position to an approximated position for clamping tissue between jaw 284 and blade 282. Drive assembly 250 provides a force-limiting feature whereby the clamping pressure applied to tissue is limited to a particular clamping pressure or particular clamping pressure range. Rotation knob 270 is rotatable in either direction to rotate elongated assembly 200 in either direction relative to handle assembly 100.

Waveguide 230 extends through inner support sleeve 220. Waveguide 230 defines a body 232 and a blade 282 extending from the distal end of body 232. Blade 282 serves as the blade of end effector 280. Waveguide 230 further includes a proximal threaded male connector 236 configured for threaded engagement within threaded female receiver 325e of nose 325b of ultrasonic horn 324 of ultrasonic transducer assembly 320 such that ultrasonic vibrations produced by ultrasonic transducer assembly 320 are transmitted along waveguide 230 to blade 282 for treating tissue clamping between blade 282 and jaw 284 or positioned adjacent to blade 282.

Continuing with reference to FIGS. 1 and 2, generator assembly 310 includes a plurality of ring contacts 364, 366, 368 surrounding ultrasonic transducer assembly 320 and disposed in slidable contact with corresponding contacts 334, 336, 338, respectively, of contact assembly 332 of ultrasonic transducer assembly 320. Thus, ring contacts 364, 366, 368 and respective contacts 334, 336, 338 connect to enable drive and/or data signals to be communicated between generator assembly 310 and piezoelectric stack 322 of ultrasonic transducer assembly 320 regardless of the rotational orientation of ultrasonic transducer assembly 320 relative to generator assembly 310. More specifically, with respect to drive signal communication, the first of electrode assemblies 330 includes at least one positive electrode disposed between the piezoelectric elements (not explicitly shown) of piezoelectric stack 322 and an electrode connector connecting the at least one positive electrode with contact 334 which, in turn, is disposed in contact with ring contact 364. The second of electrode assemblies 330 includes at least one negative electrode disposed between the piezoelectric elements 323 of piezoelectric stack 322 and an electrode connector connecting the at least one negative electrode with contact 336 which, in turn, is disposed in contact with ring contact 366. As such, a drive signal voltage may be applied from generator assembly 310 across the piezoelectric elements 323 of piezoelectric stack 322 via the positive and negative electrodes. Piezoelectric stack 322, in turn, converts the applied voltage into mechanical energy, in the form of ultrasonic vibrations, that is transmitted to ultrasonic horn 324. In other embodiments, the second of the electrode assemblies 330 is omitted and casing 340 is utilized as the negative electrode for piezoelectric stack 322.

With respect to data signal communication, contact assembly 332 may include a data chip (not explicitly shown) (or electrical connectors, with the data chip disposed within generator assembly 310) may be disposed in communication with ultrasonic horn 324 (and/or other portions of ultrasonic transducer assembly 320). The data chip, more specifically, may be a microprocessor chip or other suitable chip with sensory circuitry to detect various conditions, parameters, properties, etc. of piezoelectric stack 322, ultrasonic horn 324, and/or other portions of ultrasonic transducer assembly 320. The data chip may be configured to sense, for example, a frequency, amplitude, impedance, and/or temperature of ultrasonic horn 324 (or other portion of ultrasonic transducer assembly 320); the number of times ultrasonic transducer assembly 320 has been activated, the duration of activation ultrasonic transducer assembly 320, etc. The data chip may additionally or alternatively include a memory storing information relating to ultrasonic transducer assembly 320 such as, for example, model, serial number, manufacture date, calibration and/or testing information, manufacturer setting information, etc. In embodiments where the data chip includes sensor circuitry, the memory may also store the sensed data.

The data chip (or electrical connectors) within ultrasonic transducer assembly 320 are coupled to contact 338 of contact assembly 332 which, in turn, is disposed in contact with ring contact 368 to enable communication of data signals between ultrasonic transducer assembly 320 and ultrasonic generator assembly 310.

Ultrasonic horn 324 includes a body 325a disposed within casing 340 of ultrasonic transducer assembly 320 and a nose 325b extending distally from body 325a externally of casing 340 of ultrasonic transducer assembly 320. A proximal collar 325c is disposed between body 325a and nose 325b to facilitate formation of a hermetic seal between casing 340 and ultrasonic horn 324, as detailed below. Proximal collar 325c may be disposed at or near a nodal point along ultrasonic horn 324. Nose 325b of ultrasonic horn 324 defines a distal threaded female receiver 325e configured to enable releasable threaded engagement of waveguide 230 with ultrasonic horn 324. Ultrasonic horn 324 may be formed from a metal, e.g., titanium, aluminum, stainless steel, an amorphous metal, etc., or other suitable material(s).

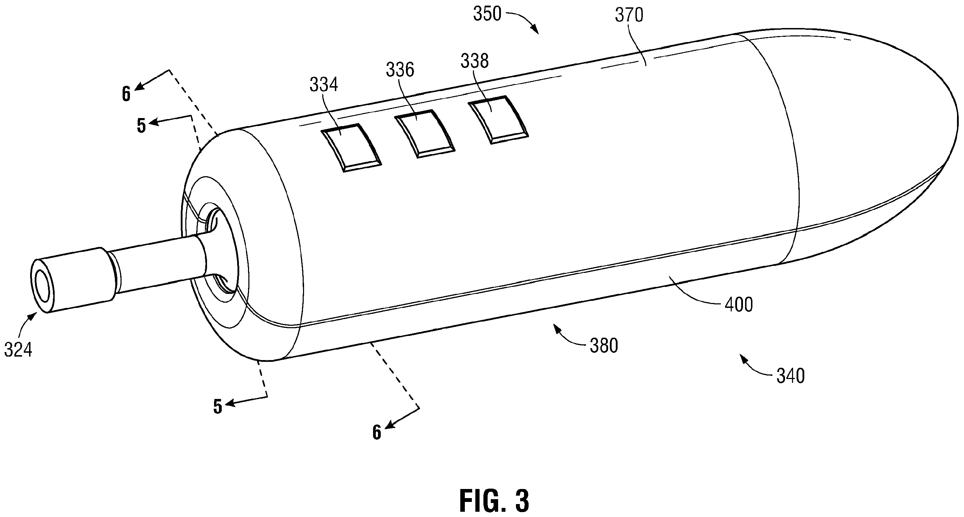

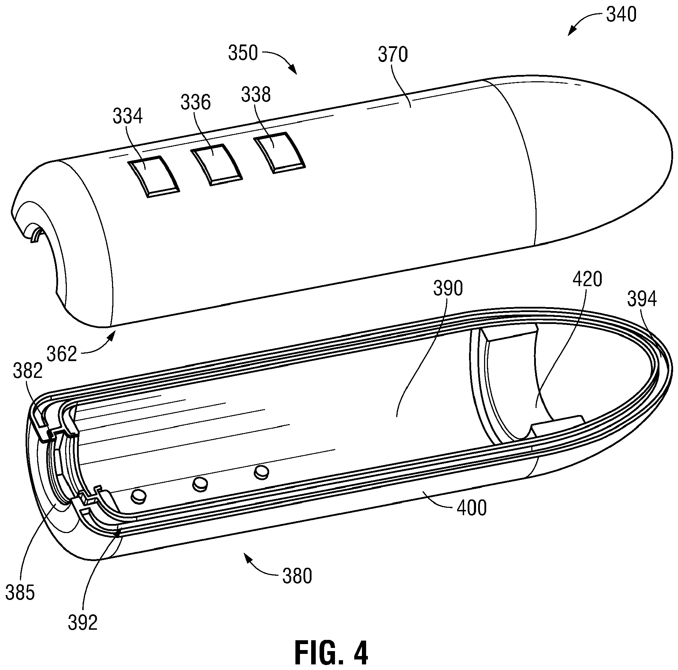

Referring to FIGS. 3-6, ultrasonic transducer assembly 320 includes a casing 340 having a first portion 350 and a second portion 380 configured to be fused, e.g., ultrasonically welded to one another. Casing 340 may be formed from a high performance thermoplastic polymer material e.g., polysulfone, polyaryletherketone, stabilized polypropylene, nylon, polyethylene, polyester, teflon, epoxy, or other suitable electrically-insulative material(s). In other configurations casing 340 or a portion thereof may be electrically-conductive.

First portion 350 of casing 340 includes a first inner surface 360, a first outer surface 370, and a first opening 355 defined at a distal end portion of first portion 350 of casing 340. First portion 350 further includes a first mating surface 362 disposed about the perimeter of first portion 350 at the interface between first inner surface 360 and first outer surface 370. First mating surface 362 includes a ridge 364 disposed along first mating surface 362 of first portion 350. First outer surface 370 of first portion 350 includes pass-through electrical contact(s) or contact(s) 334, 336, 338 extending therethrough and into the interior of casing 340 to enable drive and/or data signals to be communicated between generator assembly 310 and piezoelectric stack 322 of ultrasonic transducer assembly 320 (see FIG. 2). First portion 350 further includes a first seal member 357 surrounding first opening 355 and overmolded into a groove defined within first portion 350 of casing 340 that surrounds first opening 355. First seal member 357 is configured to receive a portion of proximal collar 325c of ultrasonic horn 324 to facilitate formation of a hermetic seal between casing 340 and ultrasonic horn 324.

Second portion 380 of casing 340 includes a second inner surface 390, a second outer surface 400, and a second opening 385 defined at a distal end portion of second portion 380 of casing 340. Second portion 380 further includes a second mating surface 392 disposed about the perimeter of second portion 380 at the interface between second inner surface 390 and second outer surface 400. Second mating surface 392 includes a trough 394 defined within second mating surface 392 of second portion 380 and configured to receive ridge 364 of first mating surface 362. In some instances, instead of the first outer surface 370 including pass-through electrical contact(s) or contacts 334, 336, 338, the second outer surface 390 of second portion 380 may include plurality of pass-through electrical contact(s) or contacts 334, 336, 338 extending therethrough and into the interior of casing 340. Second portion 390 further includes a second seal member 387 surrounding second opening 385 and overmolded into a groove defined within second portion 380 of casing 340 that surrounds second opening 385. Second seal member 387 is configured to receive a portion of proximal collar 325c of ultrasonic horn 324 to facilitate formation of a hermetic seal of casing 340 with ultrasonic horn 324. Second inner surface 390 (and/or first inner surface 360) may further include a bumper 420 disposed thereon towards a proximal end portion of casing 340 to provide support for piezoelectric stack 322 and/or support components thereof, e.g., the distal end mass and/or distal bolt (or other securement structure(s)) that compress and secure the piezoelectric stack 322 against the ultrasonic horn 324 (see FIG. 2).

Referring also to FIG. 2, to assembly ultrasonic transducer assembly 320, the inner assembly is pre-assembled, e.g., the electrodes of electrode assemblies 330 are disposed between the piezoelectric elements 323 of piezoelectric stack 322 and piezoelectric stack 322 is secured to ultrasonic horn 324 under pre-compression, e.g., using a stress rod, end mass(es), and a bolt or other suitable components (not show). Thereafter, the inner assembly is disposed within second portion 380 of casing 340 and first portion 350 is disposed about the inner assembly in assignment with the second portion 380 (or vice versa) such that first and second mating surfaces 362, 392 contact or approximate one another with ridge 364 of first mating surface 362 disposed at least partially within trough 394 of second mating surface 392, and such that proximal collar 325c of ultrasonic horn 324 extends into first and second seal members 357, 387. Thereafter, first and second portions 350, 380 are fused, e.g., ultrasonically welded, to one another about the perimeters thereof such that ridge 364 of first mating surface 362 melts into trough 394 of second mating surface 392 thus binding first portion 350 and second portion 380 together and hermetically sealing casing 340 about its perimeter between first mating surface 362 and second mating surface 392. The fusion of first portion 350 and second portion 380 compresses first seal member 357 and second seal member 387 around proximal collar 325c of ultrasonic horn 324 to hermetically seal casing 340 about ultrasonic horn 324.

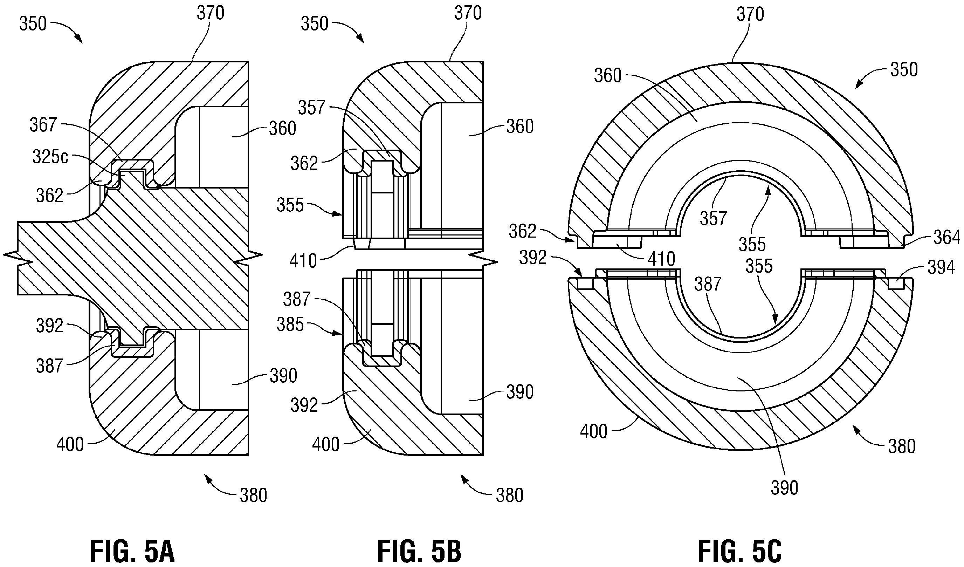

Additionally, and/or alternatively, with momentary reference to FIG. 5A-5C, a perimeter seal 410 may be disposed on, e.g., overmolded onto, first portion 350 of casing 340 to extend towards second portion 380 of casing 340. Perimeter seal 410 is disposed inwardly of ridge 364 along first mating surface 362 of first portion 350 and/or an additional or alternative perimeter seal 410 may extend from second portion 380 towards first portion 350 and may be disposed inwardly of trough 394 along second mating surface 392 of second portion 380. In some instances, perimeter seal 410 may be integrally formed with first seal member 357 and/or second seal member 387. Perimeter seal 410 of first mating surface 362 and/or second mating surface 392 is configured to compress against second mating surface 392 and/or first mating surface 362, respectively, to hermetically seal casing 340 between first mating surface 362 and second mating surface 392, even in the event of breakage, leakage, or other failure of the fusion, e.g., ultrasonic weld, therebetween.

The above-detailed hermetic sealing of casing 340 to ultrasonic horn 324 ensures that transducer assembly 320 is capable of withstanding multiple rounds of sterilization, e.g., autoclave sterilization.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

The invention may be described by reference to the following numbered paragraphs:-
1. A casing of an ultrasonic transducer assembly, comprising:
   a first portion including a ridge disposed on a first mating surface of the first portion;
   a second portion including a trough defined within a second mating surface of the second portion and configured to receive the ridge of the first portion; and
   at least one seal member extending from at least one of: the first mating surface of the first portion inwardly of the ridge or the second mating surface of the second portion inwardly of the trough,
   wherein the first mating surface of the first portion and the second mating surface of the second portion are fused to one another and wherein the fusion and the at least one seal member establish a hermetic seal between the first and second mating surfaces.
2. The casing according to paragraph 1, wherein the at least one seal member is engaged with the one of the first mating surface or the second mating surface via overmolding.
3. The casing according to paragraph 1, wherein the at least one seal member includes one seal member extending from the first mating surface and another seal member extending from the second mating surface.
4. The casing according to paragraph 1, wherein the first portion further includes a first distal opening configured to receive a first portion of an ultrasonic horn of an ultrasonic transducer assembly and wherein the second portion further incudes a second distal opening configured to receive a second portion of the ultrasonic horn.
5. The casing according to paragraph 4, further comprising a first distal seal member surrounding the first distal opening and a second distal seal member surrounding the second distal opening.
6. The casing according to paragraph 5, wherein the first and second distal seal members are engaged with the first and second portions, respectively, via overmolding.
7. The casing according to paragraph 5, wherein one of the first distal seal member or the second distal seal member is integrally formed with the seal member.
8. The casing according to paragraph 5, wherein the first and second distal seal members are configured to establish a hermetic seal about the ultrasonic horn.
9. The casing according to paragraph 1, wherein at least one of the first portion or the second portion further includes at least one pass-through electrical contact.
10. The casing according to paragraph 1, wherein at least one of the first portion or the second portion further includes a bumper disposed therein towards a proximal end thereof to support a proximal end portion of an ultrasonic transducer assembly.
11. An ultrasonic transducer assembly of an ultrasonic surgical instrument, comprising
   a piezoelectric stack;
   an ultrasonic horn secured to and extending distally from the piezoelectric stack, the ultrasonic horn including a body and a nose extending distally from the body; and
   a casing disposed about the piezoelectric stack and at least a portion of the body of the ultrasonic horn with the nose extending distally from the casing, the casing including:
      a first portion including a ridge disposed on a first mating surface of the first portion; and
      a second portion including a trough defined within a second mating surface of the second portion and configured to receive the ridge of the first portion; and
      at least one seal member extending from at least one of: the first mating surface of the first portion inwardly of the ridge or the second mating surface of the second portion inwardly of the trough,
      wherein the first mating surface of the first portion and the second mating surface of the second portion are fused to one another and wherein the fusion and the at least one seal member establish a hermetic seal between the first and second mating surfaces.
12. The ultrasonic transducer assembly according to paragraph 11, wherein the at least one seal member is engaged with the one of the first mating surface or the second mating surface via overmolding.
13. The ultrasonic transducer assembly to paragraph 11 , wherein the at least one seal member includes one seal member extending from the first mating surface and another seal member extending from the second mating surface.
14. The ultrasonic transducer assembly to paragraph 11, wherein the first portion further includes a first distal opening configured to receive a first portion of the ultrasonic horn and wherein the second portion further incudes a second distal opening configured to receive a second portion of the ultrasonic horn.
15. The ultrasonic transducer assembly to paragraph 11, wherein the first overmold and the second overmold establishes a hermetic seal.
16. The ultrasonic transducer assembly to paragraph 14, further comprising a first distal seal member surrounding the first distal opening and a second distal seal member surrounding the second distal opening.
17. The ultrasonic transducer assembly to paragraph 16, wherein the first and second distal seal members are engaged with the first and second portions, respectively, via overmolding.
18. The ultrasonic transducer assembly to paragraph 16, wherein one of the first distal seal member or the second distal seal member is integrally formed with the seal member.
19. The ultrasonic transducer assembly to paragraph 16, wherein the first and second distal seal members are configured to establish a hermetic seal about the ultrasonic horn.
20. The ultrasonic transducer assembly to paragraph 11, wherein at least one of the first portion or the second portion further includes a bumper disposed therein towards a proximal end thereof to support the piezoelectric stack.

## Claims

1. A casing of an ultrasonic transducer assembly, comprising:
a first portion including a ridge disposed on a first mating surface of the first portion;
a second portion including a trough defined within a second mating surface of the second portion and configured to receive the ridge of the first portion; and
at least one seal member extending from at least one of: the first mating surface of the first portion inwardly of the ridge or the second mating surface of the second portion inwardly of the trough,
wherein the first mating surface of the first portion and the second mating surface of the second portion are fused to one another and wherein the fusion and the at least one seal member establish a hermetic seal between the first and second mating surfaces.

2. The casing according to claim 1, wherein the at least one seal member is engaged with the one of the first mating surface or the second mating surface via overmolding.

3. The casing according to claim 1 or 2, wherein the at least one seal member includes one seal member extending from the first mating surface and another seal member extending from the second mating surface.

4. The casing according to claim 1, 2 or 3 wherein the first portion further includes a first distal opening configured to receive a first portion of an ultrasonic horn of an ultrasonic transducer assembly and wherein the second portion further incudes a second distal opening configured to receive a second portion of the ultrasonic horn.

5. The casing according to claim 4, further comprising a first distal seal member surrounding the first distal opening and a second distal seal member surrounding the second distal opening.

6. The casing according to claim 5 wherein the first and second distal seal members are engaged with the first and second portions, respectively, via overmolding.

7. The casing according to claim 5 or 6 wherein at least one of the first distal seal member or the second distal seal member is integrally formed with the seal member.

8. The casing according to claim 5, 6 or 7 wherein the first and second distal seal members are configured to establish a hermetic seal about the ultrasonic horn.

9. The casing according to any preceding claim, wherein at least one of the first portion or the second portion further includes at least one pass-through electrical contact.

10. The casing according to any preceding claim, wherein at least one of the first portion or the second portion further includes a bumper disposed therein towards a proximal end thereof to support a proximal end portion of an ultrasonic transducer assembly.

11. An ultrasonic transducer assembly of an ultrasonic surgical instrument, comprising
a piezoelectric stack;
an ultrasonic horn secured to and extending distally from the piezoelectric stack, the ultrasonic horn including a body and a nose extending distally from the body; and
a casing as claimed in any preceding claim disposed about the piezoelectric stack and at least a portion of the body of the ultrasonic horn with the nose extending distally from the casing.

12. The ultrasonic transducer assembly to claim 11, wherein the first overmold and the second overmold establishes a hermetic seal.
